# EUROPEAN PATENT SPECIFICATION

(11) **EP 2 155 001 B1**
(45) Date of publication and mention of the grant of the patent: **08.06.2016**
(21) Application number: 08741608.7
(22) Date of filing: 14.04.2008
(51) Int. Cl.: A23L 3/3472, C07H 15/18, A61K 36/889, A23D 9/007, A23L 3/3544, A23L 3/3481

(54) **PALM PHENOLICS AND FLAVONOIDS AS POTENT BIOLOGICAL AND CHEMICAL ANTIOXIDANTS FOR APPLICATIONS IN FOODS AND EDIBLE OILS.**
PALMPHENOLE UND FLAVONOIDE ALS HOCHWIRKSAME BIOLOGISCHE UND CHEMISCHE ANTIOXIDANTIEN FÜR ANWENDUNGEN BEI NAHRUNGSMITTELN UND SPEISEÖLEN
PHÉNOLIQUES ET FLAVONOÏDES DE PALME UTILES EN TANT QU'ANTIOXYDANTS BIOLOGIQUES ET CHIMIQUES POUR DES APPLICATIONS DANS DES ALIMENTS ET DES HUILES ALIMENTAIRES

(30) Priority: 12.04.2007 MY 0700567
(43) Date of publication of application: 24.02.2010
(73) Proprietor: Malaysian Palm Oil Board, Selangor Darul Ehsan (MY)
(72) Inventor: TAN, Yew Ai, 56000, Kuala Lumpur (MY); RAVIGADEVI a/p Sambanthmurthi, Selangor Darul Ehsan (MY); KALYANA, Sundram a/l Manickam, Selangor Darul Ehsan (MY); MOHD, Basri, Bin Wahid, Selangor Darul Ehsan (MY)
(74) Representative: Lloyd, John Scott
(86) International application number: PCT/MY2008/000031
(87) International publication number: WO 2008/127086

(56) References cited:
- WO-A1-2006/005986
- US-A- 5 084 289
- US-A- 5 084 293
- US-A- 5 607 707
- US-A1- 2003 031 740
- US-B2- 7 182 971
- BALASUNDRAM N ET AL: "Antioxidant properties of palm fruit extracts", ASIA PACIFIC JOURNAL OF CLINICAL NUTRITION, SMITH-GORDON JOURNAL, LONDON, GB, vol. 14, no. 4, 1 January 2005 (2005-01-01), pages 319-324, XP008122114, ISSN: 0964-7058
- SALTA F N ET AL: "Oxidative stability of edible vegetable oils enriched in polyphenols with olive leaf extract", FOOD SCIENCE AND TECHNOLOGY INTERNATIONAL, SAGE PUBLICATIONS, NEW YORK, NY, US, vol. 13, no. 6, 1 January 2007 (2007-01-01), pages 413-421, XP002541264, ISSN: 1082-0132, DOI: 10.1177/1082013208089563
- BALASUNDRAM N. ET AL.: 'Antioxidant properties of palm fruit extracts' ASIA PAC. J. CLIN. NUTR. vol. 14, no. 4, December 2005, pages 319 - 324, XP008122114

## Description

### FIELD OF INVENTION

This invention relates to the use of palm phenolics which include but is not limited to phenolic acids and flavonoids as natural antioxidants and food preservatives. It is also directed to a process for preparing an emulsion of palm in non-ionic substances which have both hydrophilic and lipophilic properties for use in food products and edible oils.

### BACKGROUND OF THE INVENTION

Antioxidants are substances that when present in food or in the body at low concentrations compared to that of an oxidizable substrate markedly delay or prevent the oxidation of that substrate. The role of antioxidants is to extend the shelf life of foodstuffs and to reduce wastage and nutritional losses by inhibiting and delaying oxidation. In food, antioxidants occur either as endogenous constituents or are added for enhancing product quality by controlling oxidation with its deleterious consequences. The mechanism by which antioxidants protect food from oxidation is by scavenging of free radicals via donation of an electron or a hydrogen atom, or by deactivation of metal ions and singlet oxygen. The ideal food grade antioxidant should be safe and not impart colour, odour or flavour. It should also be effective at low concentrations, be easy to incorporate into foods, survive harsh processing conditions, be stable in the finished product and available at low cost. Whilst antioxidants are often added to foods to stabilize them and prevent off-flavour development, considerable interest has been expressed for their potential role as therapeutic agents. Consequently, antioxidants are of interest to both food scientists and health professionals.

Lipid peroxidation is a problem in the edible oil and food industry as well as in the human body. Excess production of oxygen radical species, particularly hydroxyl radicals, can affect lipid cell membranes to produce lipid peroxides and reactive oxygen species which are linked to a variety of diseases as well as acceleration of the aging process. Oils and fats undergo oxidative deterioration on heating and storage and antioxidants are added to increase their shelf life by retarding the development of oxidation products. The most widely used antioxidants or oxidation inhibitors are tertiary butyl hydroquinone (TBHQ), butylated hydroxanisole (BHA) and butylated hydroxytoluene (BHT). However, several countries have now restricted the use of these synthetic antioxidants because of growing evidence on their health risks. In general, natural antioxidants are preferred by consumers and are more likely to gain legislative approval more easily. An ideal antioxidant will be from a natural source, effective at an economical concentration and imparts no undesirable flavour, haze or colour to the oil. Oxidation of lipids in food affects its nutritional quality, wholesomeness, safety, colour, flavour and texture. In the past, the use of approved synthetic antioxidants in foods has proven to be an effective and economical method of inhibiting oxidation and prolonging shelf-life. However, growing consumer concerns over the use of chemicals in foods, non-synthetic antioxidants from natural food ingredients are fast gaining the attention of food manufacturers.

Naturally occurring inhibitors of oxidation in food generally originate from plant-based materials. The active components, namely phenolics, flavonoids and polyphenolics, including tocopherols, are secondary plant metabolites and are first derived from phenylalanine and in certain cases and in some plants from tyrosine. Examples of some common plant phenolic antioxidants include flavonoid compounds, cinnamic acid derivatives, coumarins and tocopherols. Several studies are in progress to identify natural antioxidants while some such as rosemary oil extract and sesamol have already been extracted from plant sources and are produced commercially.

Antioxidants can be classified according to their mode of action. Thus, antioxidants can be free-radical terminators, chelators of metal ions, or oxygen scavengers that react with oxygen in closed systems. Primary antioxidants react with high-energy lipid radicals to convert them to thermodynamically more stable products and secondary antioxidants retard the rate of chain initiation by breaking down hydroperoxides. Phenolic antioxidants are included in the category of free radical terminators.

During the extraction of crude palm oil from oil palm (*Elaeis guineensis*) fruits, a large volume of aqueous waste is generated and subsequently discarded as palm oil mill effluent (POME). Oil palm fruits contain numerous phenolic compounds and most of these water-soluble components end up in the POME. This invention relates to the preparation of a composition of palm phenolics extracted from POME, for use as antioxidants in foods and edible oils. In order to make it the water-soluble palm phenolics soluble in lipid/oil systems, the palm phenolic extract was pre-treated with surface active agents to form an emulsion.

Nagendran Balasundram et al., Asia Pac. J. Clin. Nutr. Vol. 4(4), pp 319-324 (2005), describe antioxidant properties of palm fruit extracts obtained from aqueous by-products of oil palm milling.

US-A-20030031740 describes methods for the extraction of antioxidant phenolics and flavonoids from aqueous process streams of palm oil milling. The extracts are water-soluble. Various uses of the extracts are suggested.

WO-A-2006005986 describes extraction and purification of polyphenols from olive vegetable matter.

### SUMMARY OF THE INVENTION

In a first aspect, the present invention provides a method of producing an antioxidant composition for reducing oxidative deterioration of fats and edible oils, said method comprising the steps of:
a) extracting antioxidant compounds comprising palm phenolics from the plant material obtained from palm oil mill aqueous by-products, followed by either
b) dissolving the said palm phenolics extract in a solubilising medium to form a solubilisate, and admixing this solubilisate with a surface active agent to produce said antioxidant composition, or
c) pre-dissolving said palm phenolics extract in propylene glycol without the use of other solvents

In a second aspect, the present invention provides a palm phenolic composition having antioxidant activity in fats and oils and fatty foods, wherein said composition is obtainable by a method according to the present invention.

### DETAILED DESCRIPTION OF THE INVENTION

Palm phenolics extracted from palm oil aqueous by products (reference - patent for extraction of palm phenolics from POME) is water-soluble and fat-insoluble. Such compounds can be made into effective antioxidants for water-insoluble substrates e.g. in fats by forming emulsions or pastes in non-ionic substances which have both hydrophilic and lipophilic properties e.g. surface active agents.

The present invention relates to the preparation of oil soluble antioxidants from a water-soluble extract of palm phenolics recovered from the aqueous by products of the palm oil mill or what is commonly known as palm oil mill effluent (POME). Such compounds can be made into effective antioxidants for water-insoluble substrates e.g. fats by forming emulsion or paste in non-ionic substances which have both hydrophilic and lipophilic properties *e.g.* surface active agents. By dissolving the extract containing palm phenolics in a solubilising medium, such as ethanol water and admixing this with the surface active agent, an antioxidant composition of the surface active agent and extract of the palm phenolics is produced for applications in edible oils and foods. An oil soluble extract containing the palm phenolics and flavonoid can also be directly prepared by pre-dissolving it in propylene glycol without the use of other solvents.

Another method for dissolving the water-soluble palm phenolics in oil is by solubilising the water-soluble palm phenolics in water and admixing this solubilisate into a mixture of oil solution containing lecithin. The amount of lecithin present in the oil is from about 0.1-0.5 weight percent. The oil is selected from a group consisting of vegetable oils, animal fats/oils, edible oils or blends thereof. When the oil containing the lecithin is stirred after the addition of the aqueous palm phenolics solution, reverse micelles are formed. The water-soluble palm phenolics are therefore solubilised in the oil via reverse micelles.

### EXAMPLES

### Example 1

1 g of the palm phenolics was dissolved in 12.5 ml of ethanol containing 6 ml of water. This solution was added to 12 g of Tween 80 and the mixture homogenized.

### Example 2

0.5841 g of extract of the palm phenolics was mixed with 6.5 g of propylene glycol and warmed to hasten the dissolution of the extract in the propylene glycol.

### Example 3

### Use of compositions ofpalm phenolics as prepared according to Example 1 and Exampled 2 to stabilize of refined palm olein (RPOo)

The refined palm olein (RPOo) used in this experiment had a free fatty acid content of 0.23 %, iodine value of 57.1 and peroxide value of 15 meq/kg and induction time of 18 hours. Peroxide value (PV) is a standard test for evaluating the degree to which an oil has been oxidized while the induction time measured at 110° C using a Rancimat apparatus is an accepted methodology for determining relative strengths of antioxidants.

1.39 g of palm phenolics composite of Example 1 was added to 55 g of refined palm olein in a glass sample bottle and the mixture homogenized by stirring.

1.38 g of the palm phenolics composite of Example 2 was added to 55 g of refined palm olein in a glass sample bottle and the mixture homogenized by stirring.

A blank control of 55 g of refined palm olein was used as comparison.

All samples were stored in oven at 70° C for 5 weeks and were tested for peroxide value and induction times in hours using the Rancimat at 110°C at the end of the storage period.

The results presented in Table 1, demonstrate that after 5 weeks of storage at 70° C, the palm phenolic composites of Example 1 and Example 2 were able to retard the oxidation of the refined palm olein as compared with the blank control.

**Table 1: PV and rancimat induction time of refined palm olein after storage at 70 °C for 5 weeks**

| | PV (meq/kg) | Induction time (Hr) |
|---|---|---|
| Control | 88 | 0 |
| RPOo + Composite of Example 1 | 51 | 1 |
| RPOo + Composite of Example 1 | 65 | 3 |

Table 1 clearly shows that the composition of palm phenolics of Example 1 and 2 of the present invention, when added to RPOo retard the oxidative deterioration of the oil.

### Example 4

2.5 g of soybean lecithin were added to 500 g of refined palm olein (RPOo) by stirring. After homogenizing the lecithin in RPOo, 0.25 ml of 50% (w/w) aqueous palm phenolics solution was added to the oil and stirred until reverse micelles of the oil are formed uniformly.

### Example 5

### Comparison of the antioxidant efficacy of a composite of palm phenolics as prepared according to Example 4 with commercial antioxidants

The refined palm olein (RPOo) used in this example as well as in *Example 4,* had a free fatty acid content of 0.07 %, iodine value of 56 and peroxide value of 1.2 meq/kg and induction time of 25 hours.

Peroxide value (PV) is a standard test for evaluating the degree to which oil has been oxidized. Induction period is the length of time, in hours, before the rapid acceleration of oxidation. Induction periods are also known as OSI (Oxidative stability index), defined as the point of maximum change of the rate of oxidation. The determination of the OSI in this example was carried out with the test oils held at 110° C until the induction period is reached. The Oxidative Stability Instrument was used in this example as it can analyse 24 samples in one run while the Rancimat used in *Example* 3 can only test 6 samples in one run.

450 g of RPOo containing 250 mg/kg palm phenolics, prepared according to *Example 4* was transferred into a 500-ml amber-glass bottles and loosely capped. RPOo containing 1000 mg/kg Rosemary extract (sourced from KALSEC® Inc), 175 mg/kg BHA (butylhydroxy anisole) and 75 mg/kg BHT (butylhydroxy toluene) were prepared and 450 g of each of the antioxidant fortified RPOo were stored in loosely capped 500-ml amber-coloured glass bottles. The contents of BHA and BHT in RPOo were so chosen because these are the maximum levels allowed CODEX Stan 19-1981 for fats and oils. A 450g of RPOo without addition of any additive was used as the control blank.

All samples were stored in loosely capped 500-ml amber-coloured glass bottles to minimize exposure to light while handling the samples before, during and after the aging process. The Oven Storage Test for Accelerated Aging of Oils (AOCS Recommended Practice Cg 5-97) was used to measure the stability of test oils by aging/storing the test oils in an oven at 60° C, in the dark, to exclude photo-oxidation. Although separate storage containers are preferred for the removal of aliquots for each sampling time, aliquots from the same container were taken in example because amounts removed were small and did not affect the surface volume ratio significantly. To ensure uniform transfer of heat from the oven to the samples, placement of the bottles of samples in the oven was systematically changed after each weekly sampling.

The results presented in Table 2 and Table 3, demonstrate that after 3 weeks of storage at 60°C, the palm phenolic composite of *Example 4* was able to retard the oxidation of RPOo as compared with the blank control. Also demonstrated in Table 2 and Table 3, is the efficacy of the palm phenolic composite as an antioxidant for stabilizing oil, in comparison with efficacies of commercially available rosemary extract, BHA and BHT.

**Table 2: Change in PV of refined palm olein fortified with different antioxidants on storage at 60 °C in the dark for 3 weeks (21 days)**

| Sample | Before storage | After 7 days | After 14 days | After 21 days |
|---|---|---|---|---|
| Control | 1.2 | 9.4 | 19.7 | 29.3 |
| RPOo + 250 mg/kg palm phenolics | 1.1 | 2.4 | 3.7 | 6.9 |
| RPOo + 1000 mg/kg rosemary extract | 1.1 | 5.0 | 6.2 | 9.3 |
| RPOo + 175 mg/kg BHA | 1.5 | 7.3 | 16.4 | 27.9 |
| RPOo + 75 mg/kg BHT | 1.2 | 7.4 | 9.1 | 16.1 |

**Table 3: Change in induction time of refined palm olein fortified with different antioxidants on storage at 60 °C in the dark for 3 weeks (21 days)**

| Sample | Before storage | After 7 days | After 14 days | After 21 days |
|---|---|---|---|---|
| Control | 25 | 23 | 18 | 13 |
| RPOo + 250 mg/kg palm phenolics | 26 | 26 | 25 | 23 |
| RPOo + 1000 mg/kg rosemary extract | 31 | 30 | 27 | 24 |
| RPOo + 175 mg/kg BHA | 27 | 24 | 20 | 18 |
| RPOo + 75 mg/kg BHT | 28 | 26 | 24 | 21 |

## Claims

1. A method of producing an antioxidant composition for reducing oxidative deterioration of fats and edible oils, said method comprising the steps of:
a) extracting antioxidant compounds comprising palm phenolics from the plant material obtained from palm oil mill aqueous by-products, followed by either
b) dissolving the said palm phenolics extract in a solubilising medium to form a solubilisate, and admixing this solubilisate with a surface active agent to produce said antioxidant composition, or
c) pre-dissolving said palm phenolics extract in propylene glycol without the use of other solvents

2. The method as claimed in claim 1, wherein said solubilising medium in step (b) is ethanol water.

3. The method as claimed in claim 1, wherein said surface active agent in step (b) is a non-ionic surfactant.

4. The method as claimed in claim 1, wherein step (b) comprises solubilising the water-soluble palm phenolics in water and admixing this solubilisate into a mixture of oil solution containing lecithin; wherein when the oil containing the lecithin is stirred after the addition of the aqueous palm phenolics solution, reverse micelles are formed; wherein the water-soluble palm phenolics are therefore solubilised in the oil via reverse micelles.

5. The method as claimed in Claim 4, wherein amount of lecithin present in the oil is from 0.1-0.5 weight percent.

6. The method as claimed in Claim 4, wherein the oil is selected from a group consisting of vegetable oils, animal fats/oils, edible oils or blends thereof.

7. A palm phenolic composition having antioxidant activity in fats and oils and fatty foods, wherein said composition is obtainable by a method according to any preceding claim.

## Patentansprüche

1. Verfahren zum Erzeugen einer Antioxidanszusammensetzung zum Verringern des oxidativen Verderbs von Fetten und Speiseölen, wobei das Verfahren die Schritte umfasst:
a) Extrahieren von Antioxidansverbindungen, welche Palmphenole umfassen, aus einem Pflanzenmaterial, welches aus wässrigen Nebenprodukten einer Palmölmühle erhalten wurde, gefolgt entweder von
b) Lösen des Palmphenolextrakts in einem solibilisierenden Medium, um ein Solubilisat zu bilden, und Mischen dieses Solubilisats mit einem oberflächenaktiven Mittel, um die Antioxidanszusammensetzung zu erzeugen, oder
c) Vorlösen des Palmphenolextraktes in Propylenglykol ohne die Verwendung weiterer Lösungsmittel.

2. Verfahren nach Anspruch 1, wobei das solubilisierende Medium in Schritt (b) Ethanol-Wasser ist.

3. Verfahren nach Anspruch 1, wobei das oberflächenaktive Mittel in Schritt (b) ein nichtionisches Tensid ist.

4. Verfahren nach Anspruch 1, wobei Schritt (b) ein Solubilisieren der wasserlöslichen Palmphenole in Wasser und ein Beimengen dieses Solubilisats in eine Mischung einer Öllösung enthaltend Lecithin umfasst; wobei reverse Micellen gebildet werden, wenn das Öl enthaltend das Lecithin nach der Zugabe der wässrigen Palmphenollösung gerührt wird; wobei die wasserlöslichen Palmphenole somit über reverse Micellen in dem Öl solubilisiert werden.

5. Verfahren nach Anspruch 4, wobei die im Öl vorliegende Menge an Lecithin 0,1 - 0,5 Gewichtsprozent beträgt.

6. Verfahren nach Anspruch 4, wobei das Öl ausgewählt ist aus einer Gruppe bestehend aus pflanzlichen Ölen, tierischen Fetten/Ölen, Speiseölen oder Mischungen davon.

7. Eine Palmphenolzusammensetzung mit einer antioxidativen Aktivität in Fetten und Ölen und fetthaltigen Lebensmitteln, wobei die Zusammensetzung mittels eines Verfahrens gemäß einem der vorherigen Ansprüche erhältlich ist.

## Revendications

1. Procédé de production d'une composition antioxydante permettant de réduire la détérioration oxydative des graisses et des huiles comestibles, ledit procédé comprenant les étapes suivantes :
a) l'extraction de composés antioxydants comprenant des composés phénoliques de palme à partir du matériel végétal obtenu à partir de sous-produits aqueux d'huilerie de palme, ce qui est suivi
b) soit de la dissolution dudit extrait de composés phénoliques de palme dans un milieu de solubilisation pour former un solibilisat et du mélange de ce solibilisat avec un tensioactif pour produire ladite composition antioxydante, soit de
c) la dissolution préalable dudit extrait de composés phénoliques de palme dans du propylène glycol sans utiliser d'autres solvants.

2. Procédé selon la revendication 1, dans lequel ledit milieu de solubilisation dans l'étape (b) est l'éthanol eau.

3. Procédé selon la revendication 1, dans lequel ledit tensioactif dans l'étape (b) est un tensioactif non ionique.

4. Procédé selon la revendication 1, dans lequel l'étape (b) comprend la solubilisation des composés phénoliques hydrosolubles de palme dans de l'eau et le mélange de ce solibilisat dans un mélange de solution huileuse contenant de la lécithine ; dans lequel, quand l'huile contenant la lécithine est agitée après l'ajout de la solution aqueuse de composés phénoliques de palme, des micelles inversées se forment ; dans lequel les composés phénoliques hydrosolubles de palme sont donc solubilisés dans l'huile par l'intermédiaire des micelles inversées.

5. Procédé selon la revendication 4, dans lequel la quantité de lécithine présente dans l'huile est de 0,1 à 0,5 pour cent en poids.

6. Procédé selon la revendication 4, dans lequel l'huile est choisie dans le groupe constitué par les huiles végétales, les graisses/huiles animales, les huiles comestibles ou leurs mélanges.

7. Composition de composés phénoliques de palme ayant une activité antioxydante dans les graisses et les huiles et les aliments gras, dans laquelle ladite composition peut être obtenue par un procédé selon l'une quelconque des revendications précédentes.
